# EUROPEAN PATENT APPLICATION

(11) **EP 2 092 899 A2**
(43) Date of publication of application: **26.08.2009**
(21) Application number: 09153273.9
(22) Date of filing: 20.02.2009
(51) Int. Cl.: A61B 17/15

(54) **Universal cutting guide for total knee arthroplasty intervention**

(30) Priority: 21.02.2008 IT FI20080029
(71) Applicant: I+S.R.L., 50144 Firenze (IT)
(72) Inventor: Bignozzi, Simone, 39012, MERANO (IT); Marcacci, Maurilio, 40135, BOLOGNA (IT); Nofrini, Laura, 40033, Casalecchio Di Reno (IT)
(74) Representative: Borsano, Corrado

(57) **Abstract**

A universal mini invasive cutting guide is described, that is to be utilized for femoral and tibial resections during total knee arthroplasty, both for left or right limb to be used in conjunction with a computer assisted surgery system (CAS). The guide has a curvature similar to a generic proximal tibia and distal femur.

## Description

### Field of the invention

The invention refers to the field of orthopaedics surgical instruments in orthopaedics.

State of the art

Articular prostheses and revision prostheses are implantable surgical devices that are used to substitute, totally or partially, a sick or damaged articulation in the muscolo-skeletal system of a human being or of an animal in order to restore a correct alignment and ligament balance of the lower limbs.

Total knee arthroplasty is a procedure for the substitution of the articular components of the knee damaged by trauma or pathology.

The total knee arthroplasty represents the state of the art for all advanced degenerative arthropathies that involve this joint.

As known, during surgical procedure the pathological surfaces, of the knee joint, are removed and substituted with metallic surfaces with similar shape in order to recreate the joint.

The application of mini-invasive surgery to total knee arthroplasty represents a non eludible option in the moment that the opportunity of anatomical structure preservation has been demonstrated by clinical outcomes.

The minimal invasiveness of total knee arthroplasty is granted by using smaller prostheses that reduce the bone removal, leaving it as intact as possible, as also by using tools that reduce the biological damage allowing a faster recovery.

For the insertion of prosthetic components, the surgeon, during the intervention, has to perform two bone resections in particular one on the distal femur and one on the proximal tibia. In order to perform these cuts the surgeon utilizes a cutting guide that allows guiding the saw.

At present manual surgical techniques foresee cutting guides which minimal invasiveness exclude the universal usability for left or right limb, and vice-versa. Consequently the cutting guide represents a tool that affects the invasiveness and therefore the recovery times and production costs because four different shapes according to the operated limb exist with relative accessories and supports.

Moreover it must be considered that mini-invasive surgery gets huge benefits by the use of computer assisted surgery.

In fact navigation systems allow augmenting the visual feedback of the operator, integrating images taken from real scenario with virtual structures drawn from data acquired on the patient.

From what said, the importance of having a guide that can be used both for tibial and femoral cuts, on left and right limbs and that can at the same time be compatible with computer assisted surgical systems, in order to optimize its use and preserve the application from possible problems due to mini-invasiveness, is evident.

### Summary of the invention

This invention allows to overcome the above-mentioned problems thanks to a cutting guide able to be utilized both for femoral and tibial cut on the right or the left limb.

Advantages and characteristics of the device will be described below in details.

Short description of the figures:
Figure 1 represents an isometric view of the cutting guide;
Figure 2 represents the upper view of the cutting guide;
Figure 3a represents the exploded view of the cutting guide with the support pin 8 and the handle 7 for femoral resection on a left limb, in case of medial access;
Figure 3b represents the view of the cutting guide on the femur on a left limb with the support pin 8 and the handle 7, in case of medial access;
Figure 4a represents the exploded view of the cutting guide with the support pin 8 and the handle 7 for tibial resection on a left limb, in case of medial access;
Figure 4b represents the view of the cutting guide on the tibia on a left limb with the support pin 8 and the handle 7, in case of medial access;

### Detailed description of the invention

As shown in the above shortly described figures the cutting guide, that is the matter of the present invention, includes a rigid body 12 with curved shape and with a curvature similar to a generic proximal tibia and distal femur in order to allow its optimal use during the planned bone resection.

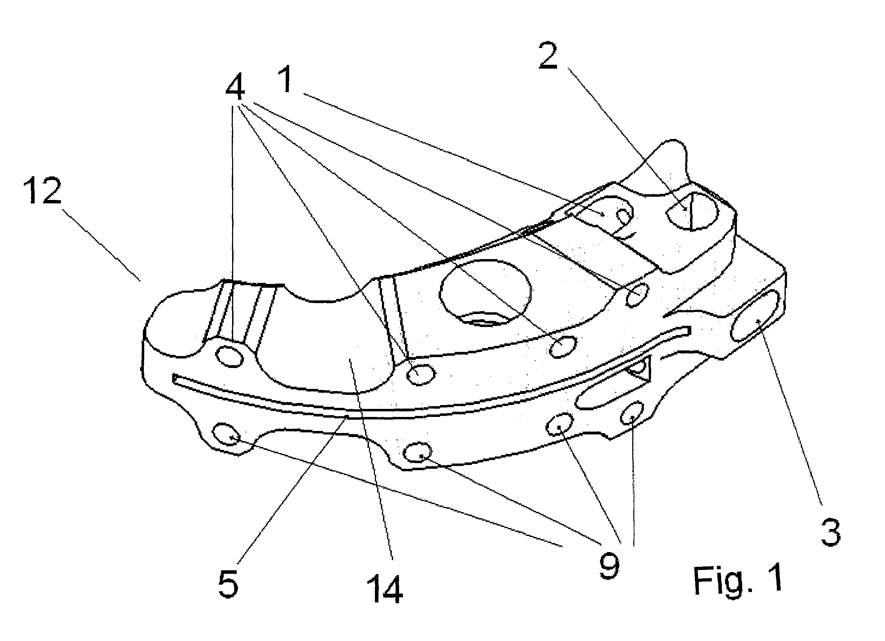

Preferably, in order to reduce the weight, the body 12 has at least a central hole 13 and the body itself is appropriately thinned where possible (see concavity 14) in order to reduce as much as possible the use of material, moreover the edges are preferably rounded (as shown in fig. 1) in order to optimize the minimal invasiveness during surgery.

The body 12 has a lateral paddle 10 that permits the positioning under the patellar tendon without damaging it allowing a correct positioning of the cutting guide for the bone resections in agreement with the principles of mini-invasiveness.

In the body 12 an open slot 5 is present positioned along the curvature of the body 12 in central position so that the body 12 is symmetrical with respect to it.

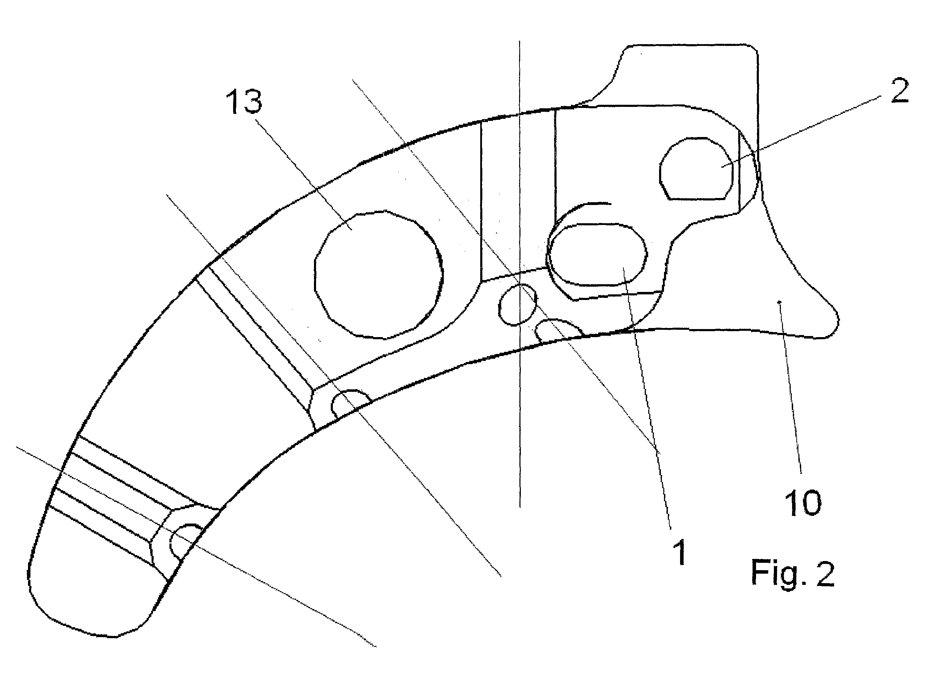

The slot 5 allows the passage and the guide of an oscillating saw (not shown in figure) that allows the bone cut. As seen in figure 2 the curved shape permits to rotate the oscillating saw in a range along the slot in order to reach easily all the zones to be resected.

Moreover the body 12 presents a hole 2 obtained perpendicular with respect to the plane containing the slot 5 and positioned near the lateral paddle 10 able to match with a support pin; the hole 2 passes therefore symmetrically on both sides of the guide allowing the use of the pin of the tibia and the femur for both the right and the left limb.

The support pin allows the surgeon to fix the cutting guide on the involved bone during the planning of the cut leaving two degrees of freedom for the final positioning of the resection plane, with a degree of freedom blocked by the pin on the bone.

According to the invention a support pin for tibia and femur is foreseen.

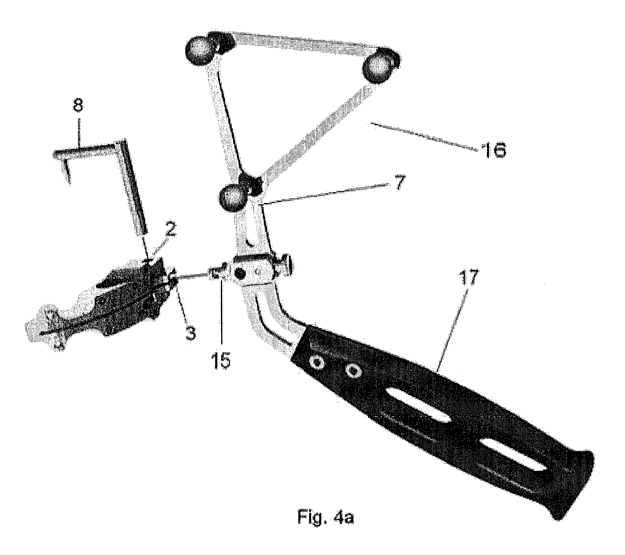

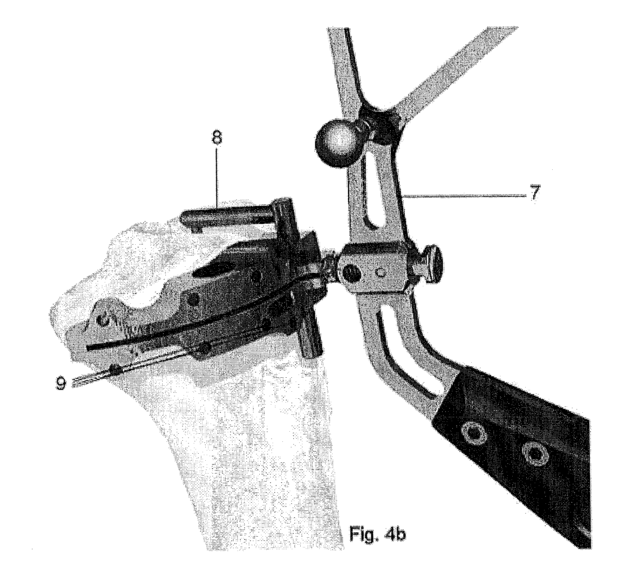

As shown in figures 4a-3a the pin 8 is preferably made of a rigid U-shaped body where an arm of this U is able to match the hole 2 and the other arm is spiked in order to enter into the bone.

The pins according to the invention as above described grant an access and a mini invasive fixation, unlike the intramedullary invasive rods normally utilized in these surgical interventions.

Symmetrically with respect to slot 5 eight holes are present (positioning the paddle 10 on the right, there are four holes 4 up and four below 8 the slot 5).

On each side of the guide the first hole permits the fixation of the slope, the two central holes are parallel and oblique, to permit the insertion of the guide while the last hole, near the paddle allows the medial fixation.

Moreover, two housings 1 and 3 are present and are able to match with complementary supporting parts of a handle 7 for the navigated guide.

The housing 1 is used to insert a handle 7 in case of femoral resection; while the housing 3 is used for the tibial resection. Consequently housings 1 and 3 are in different positions according to the positioning of the guide to the cut: the housing 1 is positioned symmetrically with respect to slot 5 in order to access from both sides, while housing 3 is positioned on the slot line.

The use of the guide, according to the invention, is extremely easy and versatile.

According to the bone and to the limb the surgeon fixes the cutting guide on the bone in the necessary position (following the indications labelled on the guide itself).

The choice of the holes to be used (on one or on the other side of the slot) for the pin insertion on the bone (not shown in figure) changes according to the surgical features to permit the fixation of the guide on the bone part that will remain after resection.

As already said the geometry of the guide, according to the invention, allows using it with a medial and lateral access.

More precisely for a medial access pins should be inserted in holes 9 for right femur and left tibia; on holes 4 for left femur and right tibia. Differently for a lateral access pins should be inserted in holes 4 for right femur and left tibia; otherwise they should be inserted in holes 9 for cutting left femur and right tibia.

The surgeon inserts the pins while blocking the positioning of the cutting guide. After performing the planned resection, the surgeon will remove the pins and the guide.

As already said the guide, according to the invention, is compatible with a navigation system, matching the more recent needing of orthopaedic surgery. This ensures the possibility to perform a cut without introducing manual errors.

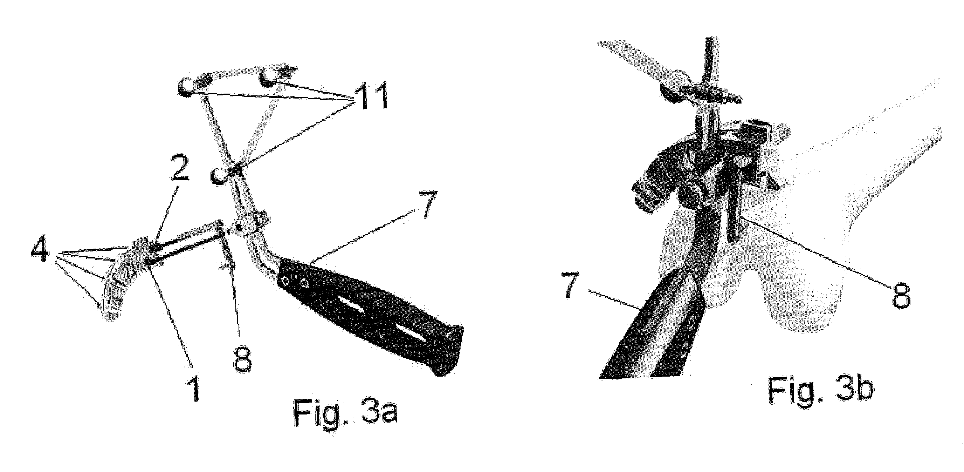

In this case for the positioning of the cutting guide the handle 7 is used (see figure 3a), this handle facilitates the use of the guide by the surgeon.

The handle 7 is constituted of an arm generally bend in an obtuse angle provided on a side by a handle 17 and on the other side by a triangular structure 16 which the vertexes can couple with three spheres 11 that are positioning markers. In the centre the handle 7 is furnished with a part 15 that can couple with the housings 1 and 3 of the cutting guide, according to the invention.

In case of computer assisted surgery, after performing the access to the knee according to the surgical technique and after anatomical landmarks acquisition that allows the planning of the resections, the navigated positioning of the cutting guide is performed.

The spheres 11 can be mounted on both sides of the handle according to the limb in order to ensure their visibility by the cameras of the navigation system in every moment, so that it is able to determine their position in the space. Since the navigation system is able to track in real time the position of the cutting guide with respect to the anatomy, it is also able to know the position of the cutting plane with respect to the planned position. During the positioning of the cutting guide, on the screen of the navigation system the planned position and the current position of the cutting guide are shown, as also the numeric and graphic indications that show the mismatch of the current positioning to the goal.

The assembling modality of the navigated handle 7 and the geometry of the guide allow the use of it in case of medial access and also in case of lateral access (so that the surgeon can choose the technique that better fits to the patient).

More precisely the housing 1 is necessary for the intervention on the right and also on the left femur (fig. 3a); it is symmetrically present on both sides of the slot allowing its use on the one or on the other side according to the intervention that has to be made. Similarly the housing 3 is necessary for the intervention on the tibia and it can be used without distinction of left or right limbs (Fig. 4a).

Following the invention the cutting guide can be realized with the more adapt materials considering the biocompatibility and the relation weight/hardness that must be as lower as possible; an example of adapt material is constituted by a alloy Cr-Co AISI 420, that besides respecting the above mentioned criteria grants a good inside resistance to the oscillating saw.

The cutting guide, according to the present invention, matches the needing of the universality of the application reducing the costs and the instrumentation, satisfies the needing of mini-invasiveness with the aim to minimize the rehabilitation times and the post-operative pain and ensures the navigation to bring safety and accuracy to the intervention.

## Claims

1. Cutting guide for total knee arthroplasty that can be used both for femoral and tibial cut on left and right limb.

2. Cutting guide according to claim 1 includes a rigid body (12) with a curvature similar to a proximal tibia and distal femur.

3. Cutting guide according to claim 2 where the body (12) presents an open slot (5) along the curvature of body (12) in the centre so that body (12) is symmetrical with respect to the slot, that permits the passage and the guide of an oscillating saw for the bone cut.

4. Cutting guide according to any of claims 1-3 where the body (12) presents, at least a central hole (13) and where the body is appropriately thinned in order to reduce as much as possible the use of material.

5. Cutting guide according to claim 4 where the body (12) presents a lateral paddle (10) that ensures the mini-invasiveness during surgery that preserves the patellar tendon.

6. Guide according to any of claims 1-5 where the body (12) presents a hole (2) perpendicular to the slot plane (5) near the lateral paddle (10) able to couple with a support pin.

7. Guide according to any of claims 1-6 where the body (12) presents eight holes symmetrically positioned with respect to slot (5), four of them up (4) and four of them below (8) the slot (5).

8. Guide according to any of claims 1-7 where the body (12) presents two housings
(1) and (3) able to couple with a handle (7) where the housing (1) is used to insert the handle (7) for femoral resection; housing (3) is used to insert handle (7) for tibial resection. Therefore housings (1) and (3) are in two different positions because of the positioning of the cutting guide according to the cut to be performed: housing (1) is symmetrical with respect to slot (5) to access from both sides, while housing (3) is at the same level of the slot (5).

9. Support pin (8) for cutting guide according to any of claims 1-8 made of a U-shaped solid body where an arm of this U is able to match the hole (2) and the other arm is spiked in order to enter into the bone.

10. Handle (7) for cutting guide according to any of claims 1-8 constituted of an arm generally bend in an obtuse angle provided on a side by a handle (17) and on the other side by a triangular structure (16) and furnished with a part (15) that can couple with the housings (1) and (3) of the cutting guide.

11. Handle according to claim 10 where the three vertexes of the structure (16) can couple with three spheres (11) which are positioning markers during navigated surgery.
